# EUROPEAN PATENT SPECIFICATION

(11) **EP 2 940 033 B1**
(45) Date of publication and mention of the grant of the patent: **25.04.2018**
(21) Application number: 13868047.5
(22) Date of filing: 18.12.2013
(51) Int. Cl.: C07K 1/14, C12P 21/02, C07K 14/435

(54) **PARTIAL PURIFICATION METHOD FOR HYDROPHILIC RECOMBINANT PROTEIN**
PARTIELLES REINIGUNGSVERFAHREN FÜR EIN HYDROPHILES REKOMBINANTES PROTEIN
PROCÉDÉ DE PURIFICATION PARTIELLE D'UNE PROTÉINE RECOMBINANTE HYDROPHILE

(30) Priority: 27.12.2012 JP 2012285392; 26.04.2013 JP 2013093926
(43) Date of publication of application: 04.11.2015
(73) Proprietor: Spiber Inc., Yamagata 997-0052 (JP)
(72) Inventor: OSAWA, Toshiaki, Tsuruoka-shi Yamagata 997-0052 (JP); MORITA, Keisuke, Tsuruoka-shi Yamagata 997-0052 (JP)
(74) Representative: Jones, Elizabeth Louise
(86) International application number: PCT/JP2013/083972
(87) International publication number: WO 2014/103847

(56) References cited:
- WO-A1-02/096931
- WO-A2-2011/133886
- JP-A- 2011 504 374
- US-A1- 2002 081 286
- US-A1- 2005 158 821
- M. Nic ET AL: "dipolar aprotic solvent" In: "IUPAC Compendium of Chemical Terminology", 24 February 2014 (2014-02-24), IUPAC, Research Triagle Park, NC, XP055299347, ISBN: 978-0-9678550-9-7 DOI: 10.1351/goldbook.D01751, * the whole document *
- CHANG N ET AL: "Protein separation and purification in neat dimethyl sulfoxide", BIOCHEMICAL AND BIOPHYSICAL RESEARCH COMMUNICATIONS, ACADEMIC PRESS INC. ORLANDO, FL, US, vol. 176, no. 3, 15 May 1991 (1991-05-15), pages 1462-1468, XP024835967, ISSN: 0006-291X, DOI: 10.1016/0006-291X(91)90451-C [retrieved on 1991-05-15]

## Description

### Technical Field

The present invention relates to a method for partially purifying a hydrophilic recombinant protein, specifically, relates to a method for partially purifying a hydrophilic recombinant protein from a host expressing a hydrophilic recombinant protein such as a recombinant spider silk protein having a hydropathy index of 0 or less.

### Background Art

Spider silk fibers are fibers having high strength and toughness, and are known to have higher strength and toughness than high-tensile steels, Nylon 6 (registered trademark) fibers, and the like. Because of these properties, spider silks have attracted attention as novel materials. By genetic recombination technologies, spider silk proteins serving as spider silk raw materials are expressed in host cells such as Escherichia coli and collected. For example, Patent Document 1 discloses production of spider dragline silk proteins such as major-ampullate dragline silk proteins of Nephila clavipes and minor-ampullate dragline silk proteins of Nephila clavipes by adopting genetic DNA technologies.

### Prior Art Document

### Patent Document

Patent Document 1: JP 1994(H06)-98771 A

### Disclosure of Invention

### Problem to be Solved by the Invention

In Patent Document 1, at the time of collecting recombinant spider dragline silk proteins present in cells by dissolving the cells to release the proteins, the spider dragline silk proteins are precipitated by repetitive processing using a solvent that dissolves cell debris but does not dissolve spider dragline silk proteins. Such a method is effective when the recombinant spider dragline silk proteins are proteins that are insoluble in water, but is not effective when the recombinant proteins are hydrophilic recombinant proteins. Therefore, simpler and easier steps have been demanded.

In order to solve the above conventional problem, it is an object of the present invention to provide a partial purification method for a hydrophilic recombinant protein that allows easy and efficient partial purification of a hydrophilic recombinant protein from a host expressing a hydrophilic recombinant protein such as a recombinant spider silk protein having a hydropathy index of 0 or less.

### Means for Solving Problem

The present invention relates to a method for partially purifying a hydrophilic recombinant protein from a host expressing a hydrophilic recombinant protein. The method includes: a solution acquisition step in which a dissolving solvent is added to the host expressing a hydrophilic recombinant protein so as to dissolve the host cell and an insoluble substance is separated therefrom so as to obtain a solution. The hydrophilic recombinant protein has a hydropathy index of 0 or less, and the dissolving solvent is an aprotic polar solvent having a dipole moment of 3.0 D or more.

In the method for partially purifying a hydrophilic recombinant protein according to the present invention, the dissolving solvent has a dipole moment of 3.0 D or more. More preferably, the dissolving solvent is one or more kinds of aprotic polar solvent selected from the group consisting of dimethyl sulfoxide, N,N-dimethylformamide, N,N-dimethylacetamide, 1,3-dimethyl-2-imidazolidone, and N-methyl-2-pyrrolidone. Preferably, the dissolving solvent is a solvent obtained by adding an inorganic salt to the aprotic polar solvent. The separation of the insoluble substance can be performed by filtration and/or centrifugation. Preferably, an addition amount (amount which is added) of the dissolving solvent is 0.5 times or more the mass of the host by volume (mL)/mass (g) ratio. Preferably, the solution is subjected to solvent substitution using an aqueous solvent. More preferably, the solution after solvent substitution is processed further by at least one selected from the group consisting of a column, filtration, and centrifugation. Preferably, the hydrophilic recombinant protein is a protein selected from the group consisting of a recombinant spider silk protein, a recombinant resilin, and a recombinant collagen.

### Effect of the Invention

According to the present invention, by using an aprotic polar solvent having a dipole moment of 3.0 D or more as a dissolving solvent, dissolving a host expressing a hydrophilic recombinant protein having a hydropathy index of 0 or less using the dissolving solvent, separating an insoluble substance therefrom and collecting a solution, it is possible to partially purify the hydrophilic recombinant protein having a hydropathy index of 0 or less from the host easily and efficiently.

Further, according to the present invention, by using a solvent obtained by adding an inorganic salt to the aprotic polar solvent as the dissolving solvent, it is possible to partially purify the hydrophilic recombinant protein such as a recombinant spider silk protein, a recombinant resilin and a recombinant collagen having a hydropathy index of 0 or less at a higher purification rate.

### Brief Description of Drawings

[FIG. 1] FIG. 1 is a photograph showing the results of SDS-polyacrylamide gel electrophoresis (SDS-PAGE) of proteins obtained in Example 1 and Comparative Example 1.
[FIG. 2] FIG. 2 is a photograph showing the results of SDS-polyacrylamide gel electrophoresis (SDS-PAGE) of proteins obtained in Examples 2-6.
[FIG. 3] FIG. 3 is a photograph showing the results of SDS-polyacrylamide gel electrophoresis (SDS-PAGE) of a protein obtained in Comparative Example 2.
[FIG. 4] FIG. 4 is a photograph showing the results of SDS-polyacrylamide gel electrophoresis (SDS-PAGE) of a protein obtained in Example 8.

### Description of the Invention

In the present invention, the hydropathy index of the protein is calculated as follows. First, the total sum of the hydropathy indices of amino acids present in the protein is calculated. Next, the total sum is divided by the number of amino acid residues present in the protein so as to calculate the average value. The average value calculated is defined as the hydropathy index of the protein. Incidentally, known hydropathy indices are used as the hydropathy indices of respective amino acids (Hydropathy index: Kyte, J. & Doolittle, R. F. (1982) A simple method for displaying the hydropathic character of a protein. J. Mol. Biol. 157, 105-132). Specifically, Table 1 below shows hydropathy indices (hereinafter, also referred to as HI) of respective amino acids.

**[Table 1]**

| Amino acid | HI | Amino acid | HI |
|---|---|---|---|
| Alanine (Ala) | 1.8 | Leucine (Leu) | 3.8 |
| Arginine (Art) | -4.5 | Lysine (Lys) | -3.9 |
| Asparagine (Asn) | -3.5 | Methionine (Met) | 1.9 |
| Aspartic acid (Asp) | -3.5 | Phenylalanine (Phe) | 2.8 |
| Cysteine (Cys) | 2.5 | Proline (Pro) | -1.6 |
| Glutamine (Gln) | -3.5 | Serine (Ser) | -0.8 |
| Glutamic acid (Glu) | -3.5 | Threonine (Thr) | -0.7 |
| Glycine (Gly) | -0.4 | Tryptophan (Trp) | -0.9 |
| Histidine (His) | -3.2 | Tyrosine (Tyr) | -1.3 |
| Isoleucine (Ile) | 4.5 | Valine (Val) | 4.2 |

The present inventors have found out that, by adding an aprotic polar solvent to a host expressing a hydrophilic recombinant protein having a hydropathy index of 0 or less to dissolve the host cell and separating an insoluble substance therefrom, the hydrophilic recombinant protein having a hydropathy index of 0 or less is collected in the supernatant and purified partially. As a result, the present inventors have reached the present invention. In other words, the present inventors have found out that, by dissolving the host cell using an aprotic polar solvent, the hydrophilic recombinant protein having a hydropathy index of 0 or less is dissolved in the aprotic polar solvent. As a result, the present inventors have reached the present invention. In the following, the hydrophilic protein refers to a hydrophilic protein having a hydropathy index of 0 or less unless otherwise specified. Further, in the following, the recombinant protein refers to a hydrophilic recombinant protein having a hydropathy index of 0 or less unless otherwise specified.

Although the aprotic polar solvent is not limited particularly, the purity of the aprotic polar solvent is preferably 90% or more, and more preferably 95% or more in terms of reducing contaminants. The aprotic polar solvent has a dipole moment of 3.0 D or more. The dipole moment is used as an index indicating the strength of the molecular polarity. The strength of the polarity increases with the dipole moment. Generally, the molecule having a dipole moment of 3.0 D or more is considered to have strong polarity. When dissolving a compound having a polarity such as protein, a solvent having high polarity is effective. Table 2 below shows dipole moments of various organic compounds (organic solvents) based on Solvent Handbook (2007) published by Kodansha Scientific Ltd. Examples of the aprotic polar solvent having a dipole moment of 3.0 D or more include dimethyl sulfoxide (DMSO), N,N-dimethylformamide (DMF), N,N-dimethylacetamide (DMA), 1,3-dimethyl-2-imidazolidone (DMI), N-methyl-2-pyrrolidone (NMP), acetonitrile, etc.

**[Table 2]**

| Organic solvent | Dipole moment (D) | Organic solvent | Dipole moment (D) |
|---|---|---|---|
| DMSO | 4.3 | NMP | 4.09 |
| DMF | 3.86 | Acetonitrile | 3.44 |
| DMA | 3.72 | Acetone | 2.69 |
| DMI | 4.05 | Isopropanol | 1.68 |

When the dissolving solvent is a solvent obtained by adding an inorganic salt to the aprotic polar solvent, the concentration of the inorganic salt is preferably, but is not particularly limited to, 0.1 M or more, more preferably 0.25 M or more, and further preferably 0.5 M or more in terms of increasing the purity (purification degree) of the recombinant protein. Further, the upper limit of the concentration of the inorganic salt in the dissolving solvent is preferably, but is not particularly limited to, saturation or lower, e.g., 2.5 M or lower.

The addition amount of the dissolving solvent is not limited particularly as long as it allows dissolution of the host. However, in terms of increasing dissolubility, the addition amount of the dissolving solvent is preferably 0.5 times or more, more preferably 0.75 times or more, and further preferably 1 time or more the mass of the host (cell) by volume (mL)/mass (g) ratio. Further, in terms of operability, the addition amount of the dissolving solvent is preferably 10 times or less, more preferably 6 times or less, and further preferably 4 times or less the mass of the host by volume (mL)/mass (g) ratio. The host (cell) may be either in a dried state or in a wet state.

The step in which the dissolving solvent is added to the host so as to dissolve the host cell is not limited particularly. However, in terms of solubility of the recombinant protein, the step is performed preferably at 20°C or higher, more preferably at 45°C or higher, and further preferably at 50°C or higher. Further, in terms of suppressing decomposition of the recombinant protein, the solution acquisition step is performed preferably at 100°C or lower, and more preferably at 95°C or lower. Further, although the solution acquisition step is not limited particularly, the step is performed preferably for 10 to 300 minutes, and more preferably for 30 to 180 minutes, for example.

In the present invention, the separation of insoluble substances is not limited particularly as long as sediments can be separated. In terms of handleability, separation by filtration and/or separation by centrifugation are preferred. The separation by filtration may be performed using, e.g., a filter paper, a filtration membrane, etc. The conditions of the centrifugation are not limited particularly. For example, the centrifugation may be performed at room temperature (20°C±5°C), 8,000 x g to 15,000 x g for 5 to 20 minutes. The separation of insoluble substances may be performed two or more times.

The recombinant protein is not limited particularly as long as it is a hydrophilic recombinant protein having a hydropathy index of 0 or less. In terms of high extraction efficiency, the recombinant protein has a hydropathy index of preferably -0.1 or less, and more preferably -0.3 or less.

Although the recombinant protein is not limited particularly, preferably, it is a protein selected from the group consisting of a recombinant spider silk protein, a recombinant resilin, and a recombinant collagen, for example.

The recombinant spider silk protein is not limited particularly as long as it is a recombinant spider silk protein derived from natural type spider silk proteins. Examples of the recombinant spider silk protein include variants, analogs, derivatives and the like of natural type spider silk proteins. In terms of excellent tenacity, the recombinant spider silk protein preferably is a recombinant spider silk protein derived from major dragline silk proteins produced in major ampullate glands of spiders. Examples of the major dragline silk proteins include major ampullate spidroins MaSp1 and MaSp2 derived from Nephila clavipes, andADF3 andADF4 derived from Araneus diadematus, etc. Further, the recombinant spider silk protein may be a recombinant spider silk protein derived from minor dragline silk produced in minor ampullate glands of spiders. Examples of the minor dragline silk proteins include minor ampullate spidroins MiSp1 and MiSp2 derived from Nephila clavipes. Further, the recombinant spider silk protein may be a recombinant spider silk protein derived from flagelliform silk proteins produced in flagelliform glands of spiders. Examples of the flagelliform silk proteins include flagelliform silk proteins derived from Nephila clavipes, etc.

Examples of the recombinant spider silk protein derived from major dragline silk proteins include a polypeptide containing two or more units of an amino acid sequence represented by the formula 1: REP1-REP2, preferably a polypeptide containing four or more units thereof, and more preferably a polypeptide containing six or more units thereof. In the recombinant spider silk protein derived from major dragline silk proteins, units of the amino acid sequence represented by the formula 1: REP1-REP2 may be the same or different from each other.

In the formula 1, the REP1 represents polyalanine. In the REP1, the number of alanine residues arranged in succession is preferably 2 or more, more preferably 3 or more, further preferably 4 or more, and particularly preferably 5 or more. Further, in the REP1, the number of alanine residues arranged in succession is preferably 20 or less, more preferably 16 or less, further preferably 14 or less, and particularly preferably 12 or less. In the formula 1, the REP2 is an amino acid sequence composed of 10 to 200 amino acid residues. The total number of glycine, serine, glutamine, proline and alanine residues contained in the amino acid sequence is 40% or more, preferably 50% or more, and more preferably 60% or more with respect to the total number of amino acid residues contained therein.

In the major dragline silk, the REP1 corresponds to a crystal region in a fiber where a crystal β sheet is formed, and the REP2 corresponds to an amorphous region in a fiber where most of the parts lack regular structures and that has more flexibility. Further, the [REP1-REP2] corresponds to a repetitious region (repetitive sequence) composed of the crystal region and the amorphous region, which is a characteristic sequence of dragline silk proteins.

An example of the polypeptide containing two or more units of the amino acid sequence represented by the formula 1: REP1-REP2 is a recombinant spider silk protein derived from ADF3 having an amino acid sequence represented by SEQ ID NO: 1. The amino acid sequence represented by SEQ ID NO: 1 is an amino acid sequence from the 1st residue to the 136th residue of an amino acid sequence that is obtained by adding an amino acid sequence (SEQ ID NO: 3) composed of a start codon, an His 10-tag and an HRV3C Protease (Human rhinovirus 3C Protease) recognition site to the N-terminal of a partial amino acid sequence of ADF3 obtained from the NCBI database (NCBI Genebank Accession No.: AAC47010, GI: 1263287). An example of a gene encoding the recombinant spider silk protein derived from ADF3 having an amino acid sequence represented by SEQ ID NO: 1 is a gene composed of a base sequence represented by SEQ ID NO: 4. Further, the polypeptide containing two or more units of the amino acid sequence represented by the formula 1: REP1-REP2 may be a polypeptide that is composed of an amino acid sequence represented by SEQ ID NO: 1 in which one or a plurality of amino acids have been substituted, deleted, inserted and/or added, and that has repetitious regions composed of crystal regions and amorphous regions and has HI of 0 or less. Incidentally, a polypeptide (ADF3Kai-small-M) having the amino acid sequence represented by SEQ ID NO: 1 has HI of -0.948.

In the present invention, "one or a plurality of" refers to 1 to 40, 1 to 35, 1 to 30, 1 to 25, 1 to 20, 1 to 15, 1 to 10, or 1 or a few, for example. Further, in the present invention, "one or a flew" refers to 1 to 9, 1 to 8, 1 to 7, 1 to 6, 1 to 5, 1 to 4, 1 to 3, 1 to 2, or 1.

Further, an example of the polypeptide containing two or more units of the amino acid sequence represented by the formula 1: REP1-REP2 is a recombinant spider silk protein derived from ADF3 having an amino acid sequence represented by SEQ ID NO: 2. The amino acid sequence represented by SEQ ID NO: 2 is an amino acid sequence from the 1st residue to the 542nd residue of an amino acid sequence that is obtained by adding the amino acid sequence (SEQ ID NO: 3) composed of a start codon, an His 10-tag and an HRV3C Protease (Human rhinovirus 3C Protease) recognition site to the N-terminal of a partial amino acid sequence of ADF3 obtained from the NCBI database (NCBI Genebank Accession No.:AAC47010, GI: 1263287). An example of a gene encoding the recombinant spider silk protein derived from ADF3 having an amino acid sequence represented by SEQ ID NO: 2 is a gene composed of a base sequence represented by SEQ ID NO: 5. Further, the polypeptide containing two or more units of the amino acid sequence represented by the formula 1: REP1-REP2 may be a polypeptide that is composed of an amino acid sequence represented by SEQ ID NO: 2 in which one or a plurality of amino acids have been substituted, deleted, inserted and/or added, and that has repetitious regions composed of crystal regions and amorphous regions and has HI of 0 or less. Incidentally, a polypeptide (ADF3Kai-NN) having the amino acid sequence represented by SEQ ID NO: 2 has HI of -0.92.

Further, an example of the polypeptide containing two or more units of the amino acid sequence represented by the formula 1: REP1-REP2 is a recombinant spider silk protein derived from ADF4 having an amino acid sequence represented by SEQ ID NO: 6. The amino acid sequence represented by SEQ ID NO: 6 is an amino acid sequence from the 1st residue to the 318th residue of an amino acid sequence that is obtained by adding the amino acid sequence (SEQ ID NO: 3) composed of a start codon, an His 10-tag and an HRV3C Protease (Human rhinovirus 3C Protease) recognition site to the N-terminal of a partial amino acid sequence of ADF4 obtained from the NCBI database (NCBI Genebank Accession No.: AAC47011, GI: 1263289). An example of a gene encoding the recombinant spider silk protein derived from ADF4 having an amino acid sequence represented by SEQ ID NO: 6 is a gene composed of a base sequence represented by SEQ ID NO: 7. Further, the polypeptide containing two or more units of the amino acid sequence represented by the formula 1: REP1-REP2 may be a polypeptide that is composed of an amino acid sequence represented by SEQ ID NO: 6 in which one or a plurality of amino acids have been substituted, deleted, inserted and/or added, and that has repetitious regions composed of crystal regions and amorphous regions and has HI of 0 or less. Incidentally, a polypeptide (ADF4Kai-W) having the amino acid sequence represented by SEQ ID NO: 6 has HI of -0.357.

An example of the recombinant spider silk protein derived from minor dragline silk proteins is a polypeptide containing an amino acid sequence represented by the formula 2: (Gly-Gly-Z)m(Gly-Ala)l(Ala)o.

In the formula 2, Z indicates any one of amino acids, in particular, it preferably is an amino acid selected from the group consisting of Ala, Tyr and Gln. Preferably, m is an integer in a range of 1 to 4,1 is an integer in a range of 0 to 4, and o is an integer in a range of 1 to 6.

Among spider silks, the minor dragline silk is wound spirally from the center of a spider net, and used as a reinforcement of the net and a yarn to wrap a captured prey. The minor dragline silk is inferior to the major dragline silk in tensile strength, but is known to have high stretchability. The reason for this is considered to be as follows: in the minor dragline silk, since many crystal regions are formed of regions where glycine and alanine are arranged alternately in succession, hydrogen bonds in the crystal regions weaken easily as compared with the major dragline silk whose crystal regions are formed only of alanine.

An example of the recombinant spider silk protein derived from flagelliform silk proteins is a polypeptide containing an amino acid sequence represented by the formula 3: (Gly-Pro-Gly-Gly-X)n. Units of the amino acid sequence represented by Gly-Pro-Gly-Gly-X may be the same or different from each other.

In the formula 3, X indicates any one of amino acids, in particular, it preferably is an amino acid selected from the group consisting of Ala, Ser, Tyr and Val. Further, n is an integer of 4 or larger, preferably an integer of 10 or larger, and more preferably an integer of 20 or larger.

An example of the polypeptide containing the amino acid sequence represented by the formula 3: (Gly-Pro-Gly-Gly-X)n is a recombinant spider silk protein derived from flagelliform silk proteins having an amino acid sequence represented by SEQ ID NO: 8. The amino acid sequence represented by SEQ ID NO: 8 is an amino acid sequence obtained by adding the amino acid sequence (SEQ ID NO: 3) composed of a start codon, an His 10-tag and an HRV3C Protease recognition site to the N-terminal of a partial sequence of flagelliform silk protein of Nephila clavipes obtained from the NCBI database (NCBI Genebank Accession No.: AAF36090, GI: 7106224), specifically, an amino acid sequence thereof from the 1220th residue to the 1662nd residue from the N-terminal that corresponds to repetitive sections and motifs in said partial sequence of flagelliform silk protein. An example of a gene encoding the recombinant spider silk protein derived from flagelliform silk proteins having an amino acid sequence represented by SEQ ID NO: 8 is a gene composed of a base sequence represented by SEQ ID NO: 9. Further, the polypeptide containing the amino acid sequence represented by the formula 3: (Gly-Pro-Gly-Gly-X)n may be a polypeptide that is composed of an amino acid sequence represented by SEQ ID NO: 8 in which one or a plurality of amino acids have been substituted, deleted, inserted and/or added, and that has repetitious regions composed of amorphous regions and has HI of 0 or less. Incidentally, a polypeptide (Flag92-short2-UU) having the amino acid sequence represented by SEQ ID NO: 8 has HI of -0.482.

Among spider silks, the flagelliform silk does not have crystal regions but has repetitious regions composed of amorphous regions, which is a major characteristic of the flagelliform silk. It is considered that since the major dragline silk and the like have repetitious regions composed of crystal regions and amorphous regions, they have both of high strength and stretchability Meanwhile, regarding the flagelliform silk, the strength is inferior to that of the major dragline silk but the stretchability is high. The reason for this is considered to be that the flagelliform silk is composed mostly of amorphous regions.

The recombinant spider silk protein preferably is a recombinant spider silk protein derived from ADF3, which is one of two principal dragline silk proteins of Araneus diadematus. The recombinant spider silk protein derived from ADF3 has advantages of basically having high strength elongation and toughness and being synthesized easily.

An example of the recombinant resilin may be a recombinant resilin derived from natural resilin. Examples of the recombinant resilin derived from natural resilin include a polypeptide containing two or more units of an amino acid sequence represented by the formula 4: REP3, preferably a polypeptide containing 4 or more units thereof, more preferably a polypeptide containing 10 or more units thereof, and further preferably a polypeptide containing 20 or more units thereof. In the formula 4, units of the amino acid sequence represented by REP3 may be the same or different from each other.

In the formula 4, REP3 indicates an amino acid sequence composed of (Ser-J-J-Tyr-Gly-U-Pro). J indicates any one of amino acids, in particular, it preferably is an amino acid selected from the group consisting of Asp, Ser and Thr. Further, U indicates any one of amino acids, in particular, it preferably is an amino acid selected from the group consisting of Pro, Ala, Thr and Ser.

An example of the polypeptide containing two or more units of the amino acid sequence represented by the formula 4: REP3 is a recombinant resilin having an amino acid sequence represented by SEQ ID NO: 10. The amino acid sequence represented by SEQ ID NO: 10 is an amino acid sequence obtained by adding an amino acid sequence (SEQ ID NO: 12) composed of a start codon and an His 6-tag to the N-terminal of a partial sequence of resilin of Drosophila melanogaster obtained from the NCBI web database (NCBI Genebank Accession No.: Q9V7U0.1, GI: 75026432), specifically, an amino acid sequence thereof from the 19th residue to the 321st residue that corresponds to repetitive sections and motifs in said partial sequence of resilin. The recombinant resilin having the amino acid sequence represented by SEQ ID NO: 10 has HI of -1.229. Further, the polypeptide containing two or more units of the amino acid sequence represented by the formula 4: REP3 may be a polypeptide that is composed of an amino acid sequence represented by SEQ ID NO: 10 in which one or a plurality of amino acids have been substituted, deleted, inserted and/or added, and that has HI of 0 or less.

Resilin is a rubber-like protein present in a specific region of the insect cuticle, and is an excellent elastic material. This substance has an elasticity of 97% and loses only 3% of storage energy as heat. It is considered that cross-linking between tyrosines in resilin brings this elasticity. The dityrosine-crosslinked resilin imparts excellent elasticity to the cuticle, and plays an important role in insect's flight system and jumping system.

An example of the recombinant collagen may be a recombinant collagen derived from natural collagens. Examples of the recombinant collagen derived from natural collagens include a polypeptide containing, in succession, 5 or more units of the amino acid sequence represented by the formula 5: REP4, preferably a polypeptide containing 8 or more units thereof, and more preferably a polypeptide containing 10 or more units thereof. In the formula 5, REP4 indicates an amino acid sequence composed of Gly-X-Y. X and Y respectively indicate any one of amino acids except Gly. In the formula 5, units of the amino acid sequence represented by REP4 may be the same or different from each other.

An example of the polypeptide containing, in succession, 10 or more units of the amino acid sequence represented by the formula 5: REP4 is a recombinant collagen having an amino acid sequence represented by SEQ ID NO: 13. The amino acid sequence represented by SEQ ID NO: 13 is an amino acid sequence obtained by adding an amino acid sequence (SEQ ID NO: 15) composed of a start codon, an His 6-tag and Ser-Ser-Gly-Ser-Ser to the N-terminal of a partial sequence of human collagen type 4 obtained from the NCBI database (NCBI Genebank Accession No.: CAA56335.1, GI: 3702452), specifically, an amino acid sequence thereof from the 301st residue to the 540th residue that corresponds to repetitive sections and motifs in said partial sequence of human collagen type 4. The recombinant collagen having an amino acid sequence represented by SEQ ID NO: 13 has HI of -0.792. Further, the polypeptide containing, in succession, 10 or more units of the amino acid sequence represented by the formula 5: REP4 may be a polypeptide that is composed of an amino acid sequence represented by SEQ ID NO: 13 in which one or a plurality of amino acids have been substituted, deleted, inserted and/or added.

Collagen is one of the proteins constituting the dermis, ligament, tendon, bone, cartilage, etc., and is the main component of extracellular matrices of multicellular animals. 30 kinds or more of human collagens have been reported. All of these collagens have a repetitious amino acid sequence represented by REP4 called a collagen-like sequence, which is a characteristic sequence of human collagens.

The recombinant protein can be produced using a host that has been transformed by an expression vector containing a gene encoding a recombinant protein. A method for producing a gene is not limited particularly, and it may be produced by amplifying a gene encoding a recombinant protein from a cell containing the desired gene by a polymerase chain reaction (PCR), etc., and cloning it, or may be synthesized chemically. A method for chemically synthesizing a gene also is not limited particularly, and it can be synthesized as follows, for example: based on information of an amino acid sequence of a recombinant protein, oligonucleotides that have been synthesized automatically with AKTA oligopilot plus 10/100 (GE Healthcare Japan Corporation) are linked by PCR, etc. At this time, in order to facilitate purification and observation of protein, a gene may be synthesized that encodes a protein composed of an amino acid sequence in which an amino acid sequence composed of an His tag has been added to the N-terminal of the amino acid sequence of the target protein.

Examples of the expression vector include a plasmid, a phage, a virus and the like that can express protein based on a DNA sequence. The plasmid-type expression vector is not limited particularly as long as it allows a target gene to be expressed in a host cell and it can amplify itself. For example, in the case of using Escherichia coli Rosetta (DE3) as a host, a pET22b(+) plasmid vector, a pCold plasmid vector and the like can be used. Among these, in terms of productivity of protein, the use of the pET22b(+) plasmid vector is preferred. Examples of the host (cell) include animal cells, plant cells, microbes and the like that can be used as protein expression systems. In terms of the production cost, the use of microbes is preferred. In terms of safety and operability, the use of Escherichia coli is more preferred.

When producing the recombinant protein using a microbe such as Escherichia coli as a host, the molecular weight of the recombinant protein is preferably 500 kDa or less, more preferably 300 kDa or less, and further preferably 200 kDa or less in terms of productivity.

The solution containing the recombinant protein may be purified further by solvent substitution. The solvent substitution can be performed by dialysis. A solvent for dialysis (dialyzing fluid) may be an aqueous solvent containing water. Examples of the aqueous solvent include water, a mixture of water and a water-miscible organic solvent, a water-soluble buffer solution, an acid aqueous solution, and a basic aqueous solution. Examples of the water include purewater, distilled water and ultrapure water. Examples of the acid aqueous solution include acid aqueous solutions such as formic acid, acetic acid and diluted hydrochloric acid. Examples of the basic aqueous solution include basic aqueous solutions such as a sodium hydroxide aqueous solution and a calcium hydroxide aqueous solution. Any known water-miscible organic solvent can be used as long as it is an organic solvent that can be mixed with water. Specific examples of the water-miscible organic solvent include alcohols such as methanol and ethanol.

When the solution containing the recombinant protein is subjected to solvent substitution using the aqueous solvent, preferably, the aprotic polar solvent is an aprotic polar solvent not having a cyclic structure. By using the aprotic polar solvent not having a cyclic structure as the dissolving solvent and using the aqueous solvent for subjecting the solution containing the recombinant protein to solvent substitution, the recombinant protein such as a recombinant spider silk protein, a recombinant resilin and a recombinant collagen with higher purity can be extracted. Examples of the aprotic polar solvent not having a cyclic structure include dimethyl sulfoxide (DMSO), N,N-dimethylformamide (DMF), and N,N-dimethylacetamide (DMA).

Further, the solution after solvent substitution may be processed further by at least one selected from the group consisting of a column, filtration, centrifugation, and the like. A gel filtration column, an ion exchange column and the like can be used as the column. A filter paper, a filtration membrane and the like can be used for the filtration. This processing further increases the purification degree (purity) of the target protein (hydrophilic recombinant protein).

### Examples

Hereinafter, the present invention will be described in further detail by way of examples. Note that the present invention is not limited to the following examples.

### (Expression of recombinant protein in Escherichia coli)

### <Gene Synthesis>

### (1) Gene Synthesis of ADF3Kai-small-M

A partial amino acid sequence of ADF3, which is one of two principal dragline silk proteins ofAraneus diadematus, was obtained from the NCBI web database (NCBI Accession No.: AAC47010, GI: 1263287), and an amino acid sequence (SEQ ID NO: 3) composed of a start codon, an His 10-tag and an HRV3C Protease (Human rhinovirus 3C Protease) recognition site was added to the N-terminal of the partial amino acid sequence of ADF3, so as to synthesize a gene encoding a polypeptide composed of an amino acid sequence (SEQ ID NO: 1), i.e., the 1st residue to the 136th residue of the resultant sequence. Consequently, a pUC57 vector to which a gene of ADF3Kai-small-M composed of a base sequence represented by SEQ ID NO: 4 had been introduced was obtained (having an Nde I site immediately upstream of the 5' terminal of the gene and an Xba I site immediately downstream of the 5'terminal thereof). Thereafter, the gene was subjected to a restriction enzyme treatment with Nde I and EcoR I, and recombined into a pET22b(+) expression vector. Thus, a pET22b(+) vector to which the gene of ADF3Kai-small-M had been introduced was obtained.

### (2) Gene Synthesis of Drosophila-Resilin-Kai

A partial sequence of resilin of Drosophila melanogaster was obtained from the NCBI web database (NCBI Genebank Accession No.: Q9V7U0.1, GI: 75026432), and a gene encoding a polypeptide composed of an amino acid sequence (SEQ ID NO: 10) was synthesized. The amino acid sequence (SEQ ID NO: 10) is an amino acid sequence obtained by adding an amino acid sequence (SEQ ID NO: 12) composed of a start codon and an His 6-tag to the N-terminal of the partial amino acid sequence of resilin from the 19th residue to the 321st residue that corresponds to repetitive sections and motifs in said sequence. Consequently, a pUC57 vector to which a gene of Drosophila-Resilin-Kai composed of a base sequence represented by SEQ ID NO: 11 had been introduced was obtained (having an Nde I site immediately upstream of the 5'terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of Drosophila-Resilin-Kai had been introduced was obtained.

### (3) Gene Synthesis of Collagen-type4-Kai

A partial sequence of human collagen type 4 was obtained from the NCBI web database (NCBI Genebank Accession No.: CAA56335.1, GI: 3702452), and a gene encoding a polypeptide composed of an amino acid sequence (SEQ ID NO: 13) was synthesized. The amino acid sequence (SEQ ID NO: 13) is an amino acid sequence obtained by adding an amino acid sequence (SEQ ID NO: 15) composed of a start codon, an His 6-tag and Ser-Ser-Gly-Ser-Ser to the N-terminal of the partial amino acid sequence of human collagen type 4 from the 301st residue to the 540th residue that corresponds to repetitive sections and motifs in said sequence. Consequently, a pUC57 vector to which a gene of Collagen-type4-Kai composed of a base sequence represented by SEQ ID NO: 14 had been introduced was obtained (having an Nde I site immediately upstream of the 5'terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of Collagen-type4-Kai had been introduced was obtained.

### <Expression of Protein>

The vectors obtained, i.e., the pET22b(+) expression vector to which the gene of ADF3Kai-small-M had been introduced, the pET22b(+) vector to which the gene of Drosophila-Resilin-Kai had been introduced and the pET22b(+) vector to which the gene of Collagen-type4-Kai had been introduced, were each transformed into Escherichia coli Rosetta (DE3). Each of the obtained single colonies was incubated for 15 hours in 2 mL of an LB culture medium containing ampicillin. Thereafter, 1.4 ml of said culture solution was added to 140 mL of an LB culture medium containing ampicillin, and incubated to an OD₆₀₀ of 3.5 under the conditions of 37°C and 200 rpm. Next, the culture solution with the OD₆₀₀ of 3.5 was added to 7L of a 2xYT culture medium containing ampicillin, together with 140 mL of 50% glucose, and incubated further to the OD₆₀₀ of 4.0. Thereafter, isopropyl-β-thiogalactopyranoside (IPTG) was added to the obtained culture solution with the OD₆₀₀ of 4.0 so that the final concentration would be 0.5 mM, thereby inducing the expression of protein. After a lapse of two hours from the addition of IPTG, the culture solution was centrifuged and bacterial cells (wet bacterial cells) were collected. By using protein solutions prepared from the bacterial cells in the culture solution before the addition of IPTG and in the culture solution after the addition of IPTG, in accordance with a general protocol, the expression of target proteins with the addition of IPTG was confirmed by SDS-PAGE (ADF3Kai-small-M: about 12.5 kDa, Drosophila-Resilin-Kai: about 28.5 kDa, Collagen-type4-Kai: about 24.5 kDa). The protein ADF3Kai-small-M had a hydropathy index of -0.948. Drosophila-Resilin-Kai had a hydropathy index of -1.29. Collagen-type4-Kai had a hydropathy index of -0.792. The Escherichia coli expressing the protein ADF3Kai-small-M, the Escherichia coli expressing the protein Drosophila-Resilin-Kai and the Escherichia coli expressing the protein Collagen-type4-Kai were stored in a freezer (-20°C).

### (Example 1)

### (Purification of Protein)

(1) To about 15 g of wet bacterial cells of the Escherichia coli expressing the protein ADF3Kai-small-M, 45 ml of DMSO containing 1 M LiCl was added, followed by dissolution at 60°C for 30 minutes. Thereafter, the resultant was centrifuged at 20°C, 11,000 x g for 10 minutes using a centrifuge ("MX-305" manufactured by TOMY SEIKO Co., Ltd.), and the supernatant (solution) was collected.
(2) By means of dialysis, the collected solution was subjected to solvent substitution of DMSO with water. A dialyzing fluid (water) was replaced every 6 hours. The dialysis was conducted for 48 hours in total. The obtained solution after dialysis was centrifuged at 20°C, 11,000 x g for 10 minutes using a centrifuge ("MX-305" manufactured by TOMY SEIKO Co., Ltd.), and the supernatant (solution after solvent substitution) was collected.
(3)To 40 ml of the solution after solvent substitution, 2 ml of 50 vol% Ni resin slurry (to be connected to His) was added, followed by shaking at room temperature for 1 hour. Next, the Ni resin was collected and washed 5 times with 10 ml of elution buffer (50 mM Tris, 50mM NaCl). Thereafter, 300 mM imidazole was used to elute the target protein (ADF3Kai-small-M), and the eluate was collected.

### (Comparative Example 1)

To about 0.2 g of wet bacterial cells of the Escherichia coli expressing the protein ADF3Kai-small-M, 500µl of pure water was added, followed by ultrasonic disruption to obtain an ultrasonically disrupted liquid.

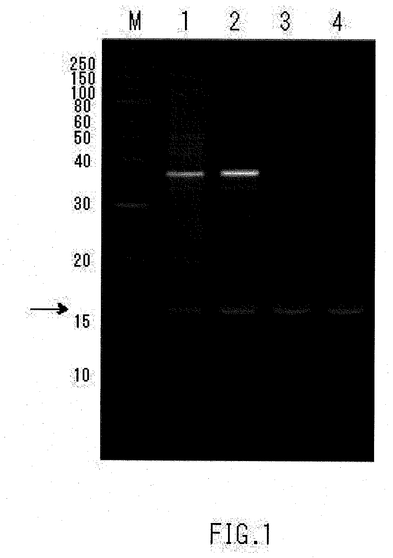

SDS-PAGE was performed using the solution, the solution after solvent substitution and the column eluate obtained in Example 1 and the ultrasonically disrupted liquid obtained in Comparative Example 1. Specifically, to 30 µL of each sample, 30 µL of a 2xSDS buffer solution (4 w/v% SDS, 20 w/v% glycerol, 10 w/v% 2-mercaptoethanol, 0.004 w/v% Bromophenol Blue, 0.125 M Tris, pH 6.8) was added, followed by heat treatment at 95°C for 10 minutes and cooling at room temperature. The heat-denatured protein solution obtained was subjected to polyacrylamide gel electrophoresis so as to check the band. After electrophoresis, the gel was stained with Oriole Fluorescent Gel Stain. FIG. 1 shows the results. The purity of the target protein (recombinant protein) was calculated by analyzing images of electrophoresis photographs of the protein after staining using analysis software ImageLab (Bio-RAD Laboratories, Inc.). Table 3 below shows the results.

**[Table 3]**

| | Sample | Purity (%) |
|---|---|---|
| Comparative Example 1 | Ultrasonically disrupted liquid | 19.6 |
| Example 1 | Solution | 32.4 |
| Example 1 | Solution after solvent substitution | 76.1 |
| Example 1 | Column eluate | 84.2 |

FIG. 1 shows the results of SDS-PAGE using the solution, the solution after solvent substitution and the column eluate obtained in Example 1 and the ultrasonically disrupted liquid obtained in Comparative Example 1. In FIG. 1, a lane M indicates a molecular-weight marker, a lane 1 indicates the protein contained in the ultrasonically disrupted liquid obtained in Comparative Example 1, and lanes 2, 3 and 4 indicate the proteins contained in the solution, the solution after solvent substitution and the column eluate obtained in Example 1, respectively. In FIG. 1, an arrow indicates the band of the protein corresponding to ADF3Kai-small-M (molecular weight: about 12.5 kDa). As can be seen from the results of FIG. 1 and Table 3, it was confirmed that the purity of ADF3Kai-small-M in the solution obtained by dissolving the Escherichia coli with the DMSO containing 1 M LiCl is higher than that of ADF3Kai-small-M in the ultrasonically disrupted liquid obtained by subjecting the Escherichia coli to ultrasonic disruption, and the target protein (ADF3Kai-small-M) was purified partially by dissolving the Escherichia coli using the aprotic polar solvent and separating an insoluble substance therefrom. Further, the purity of the target protein (ADF3Kai-small-M) was increased by subjecting the solution to solvent substitution using water. Moreover, the purity of the target protein (ADF3Kai-small-M) was increased further by processing the solution after solvent substitution using a column.

### (Example 2)

To about 15 g of wet bacterial cells of the Escherichia coli expressing the protein ADF3Kai-small-M, 45 ml of DMSO containing 1 M LiCl was added, followed by dissolution at 60°C for 30 minutes. Thereafter, the resultant was centrifuged at 20°C, 11,000 x g for 10 minutes using a centrifuge ("MX-305" manufactured by TOMY SEIKO Co., Ltd.), and the supernatant (solution) was collected.

### (Example 3)

The supernatant (solution) was collected in the same manner as in Example 2 except that DMF containing 1M LiCl was used instead of the DMSO containing 1 M LiCl.

### (Example 4)

The supernatant (solution) was collected in the same manner as in Example 2 except that DMA containing 1M LiCl was used instead of the DMSO containing 1 M LiCl.

### (Example 5)

The supernatant (solution) was collected in the same manner as in Example 2 except that NMP containing 1M LiCl was used instead of the DMSO containing 1 M LiCl.

### (Example 6)

The supernatant (solution) was collected in the same manner as in Example 2 except that DMI containing 1M LiCl was used instead of the DMSO containing 1 M LiCl.

### (Comparative Example 2)

The supernatant (solution) was collected in the same manner as in Example 2 except that isopropanol (the purity of isopropanol: 90%) containing 1M LiCl was used instead of the DMSO containing 1 M LiCl.

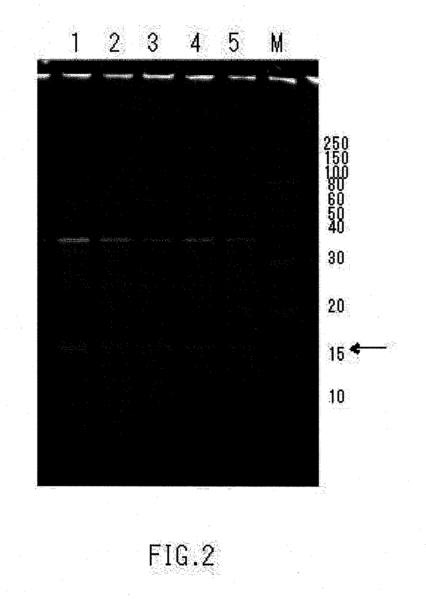

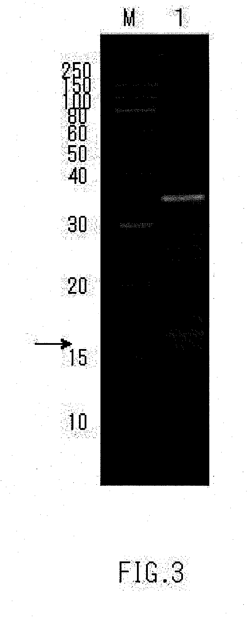

SDS-PAGE was performed as described above using the supernatants (solutions) obtained in Examples 2-6 and Comparative Example 2. After electrophoresis, the gels were stained with Oriole Fluorescent Gel Stain. FIGS. 2 and 3 show the results. The purification degree of the target protein (recombinant protein) was calculated by analyzing images of electrophoresis photographs of the protein after staining using analysis software ImageLab (Bio-RAD Laboratories, Inc.). Table 4 below shows the results.

**[Table 4]**

| | Example 2 | Example 3 | Example 4 | Example 5 | Example 6 | Comparative Example 2 |
|---|---|---|---|---|---|---|
| Dissolving solvent | DMSO | DMF | DMA | NMP | DMI | Isopropanol |
| Purity (%) | 29.9 | 23.0 | 26.3 | 22.2 | 21.6 | 9.6 |

In FIG. 2, a lane M indicates a molecular-weight marker, and lanes 1, 2, 3, 4 and 5 correspond to Examples 2, 3, 4, 5 and 6, respectively. In FIG. 2, an arrow indicates the band of the protein corresponding to ADF3Kai-small-M (molecular weight: about 12.5 kDa). It was found from FIG. 2 that the hydrophilic recombinant protein having a hydropathy index of 0 or less could be purified partially by dissolving the Escherichia coli expressing the hydrophilic recombinant protein having a hydropathy index of 0 or less using the aprotic polar solvent and separating an insoluble substance therefrom. In FIG. 3, a lane M indicates a molecular-weight marker, and a lane 1 corresponds to Comparative Example 2. In FIG. 3, an arrow indicates the band of the protein corresponding to ADF3Kai-small-M (molecular weight: about 12.5 kDa).

As can be seen from the results of Table 4 above, the purity of the target recombinant spider silk protein that had been purified partially in the Examples using the aprotic polar solvent such as DMSO was 2.2 to 3.1 times the purity of the target recombinant spider silk protein that had been purified partially in Comparative Example using isopropanol, i.e., a protic polar solvent. By using the aprotic polar solvent such as DMSO, the hydrophilic recombinant protein having a hydropathy index of 0 or less could be purified partially.

### (Example 7)

To about 0.3 g of wet bacterial cells of the Escherichia coli expressing the protein Drosophila-Resilin-Kai, 1 ml of DMSO containing 1 M LiCl was added, followed by standing at 60°C for 30 minutes for dissolution. Thereafter, the resultant was centrifuged at 20°C, 11,000 x g for 5 minutes using a centrifuge ("MX-305" manufactured by TOMY SEIKO Co., Ltd.), and the supernatant (solution) was collected.

### (Comparative Example 3)

To about 0.3 g of wet bacterial cells of the Escherichia coli expressing the protein Drosophila-Resilin-Kai, 1 ml of a Tris buffer solution (50 mM Tris, 100 mM NaCl, pH 7.0) was added, followed by ultrasonic disruption to obtain an ultrasonically disrupted liquid.

SDS-PAGE was performed as described above using the supernatant (solution) obtained in Example 7 and the ultrasonically disrupted liquid obtained in Comparative Example 3. After electrophoresis, the gels were stained with Oriole Fluorescent Gel Stain. The purification degree of the target protein (Drosophila-Resilin-Kai) was calculated by analyzing images of electrophoresis photographs of the protein after staining using analysis software ImageLab (Bio-RAD Laboratories, Inc.). The purification degree of the target protein (Drosophila-Resilin-Kai) in Example 7 was 14.5%, whereas the purification degree of the target protein (Drosophila-Resilin-Kai) in Comparative Example 3 was 8.0%.

### (Example 8)

To about 0.1 g of dried bacterial cells of the Escherichia coli expressing the protein Collagen-type4-Kai, 1 ml of DMSO containing 1 M LiCl was added, followed by standing at 60°C for 30 minutes for dissolution. Thereafter, the resultant was centrifuged at 20°C, 11,000 x g for 5 minutes using a centrifuge ("MX-305" manufactured by TOMY SEIKO Co., Ltd.), and the supernatant (solution) was collected.

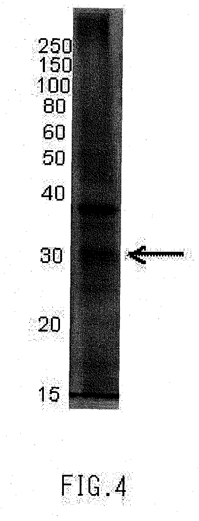

SDS-PAGE was performed as described above using the supernatant (solution) obtained in Example 8. After electrophoresis, the gel was stained with Coomassie Brilliant Blue (CBB). FIG. 4 shows the results.

In FIG. 4, an arrow indicates the band of the protein corresponding to Collagen-type4-Kai (molecular weight: about 24.5 kDa). As a result of analyzing images of electrophoresis photographs of the protein after staining using analysis software ImageLab (Bio-RAD Laboratories, Inc.), the purification degree of the target protein (Collagen-type4-Kai) was 11.6%.

### Industrial Applicability

The present invention can be used for partially purifying a hydrophilic recombinant protein from a host expressing a hydrophilic recombinant protein having a hydropathy index of 0 or less.

### Sequence Listing Free Text

SEQ ID NOS: 1, 2, 3, 6, 8, 10, 12, 13, 15 amino acid sequences
SEQ ID NOS: 4, 5, 7, 9, 11, 14 base sequences

## Claims

1. A method for partially purifying a hydrophilic recombinant protein from a host expressing a hydrophilic recombinant protein,
the method comprising a solution acquisition step in which a dissolving solvent is added to the host expressing a hydrophilic recombinant protein so as to dissolve the host cell and an insoluble substance is separated therefrom so as to obtain a solution,
wherein the hydrophilic recombinant protein has a hydropathy index of 0 or less, and
the dissolving solvent is an aprotic polar solvent having a dipole moment of 3.0 D or more.

2. The method for partially purifying a hydrophilic recombinant protein according to claim 1, wherein the dissolving solvent is one or more kinds of aprotic polar solvent selected from the group consisting of dimethyl sulfoxide, N,N-dimethylformamide, N,N-dimethylacetamide, 1,3-dimethyl-2-imidazolidone, and N-methyl-2-pyrrolidone.

3. The method for partially purifying a hydrophilic recombinant protein according to claim 1 or 2, wherein the dissolving solvent is a solvent obtained by adding an inorganic salt to the aprotic polar solvent.

4. The method for partially purifying a hydrophilic recombinant protein according to any one of claims 1 to 3, wherein the separation of the insoluble substance is performed by filtration and/or centrifugation.

5. The method for partially purifying a hydrophilic recombinant protein according to any one of claims 1 to 4, wherein the amount of the dissolving solvent which is added is 0.5 times or more the mass of the host by volume (mL)/mass (g) ratio.

6. The method for partially purifying a hydrophilic recombinant protein according to any one of claims 1 to 5, wherein the solution is subjected to solvent substitution using an aqueous solvent.

7. The method for partially purifying a hydrophilic recombinant protein according to claim 6, wherein the solution after solvent substitution is processed further by at least one selected from the group consisting of a column, filtration, and centrifugation.

8. The method for partially purifying a hydrophilic recombinant protein according to any one of claims 1 to 7, wherein the hydrophilic recombinant protein is a protein selected from the group consisting of a recombinant spider silk protein, a recombinant resilin, and a recombinant collagen.

## Patentansprüche

1. Verfahren zum partiellen Aufreinigen eines hydrophilen rekombinanten Proteins aus einem Wirt, der ein hydrophiles rekombinantes Protein exprimiert,
wobei das Verfahren einen Lösungsgewinnungsschritt umfasst, in dem ein lösendes Lösemittel zu dem Wirt, der ein hydrophiles rekombinantes Protein exprimiert, zugesetzt wird, so dass die Wirtszelle gelöst wird und eine unlösliche Substanz davon abgetrennt wird, um eine Lösung zu erhalten,
wobei das hydrophile rekombinante Protein einen Hydropathie-Index von 0 oder weniger aufweist, und
das lösende Lösemittel ein aprotisches polares Lösemittel ist, das ein Dipolmoment von 3,0 D oder mehr aufweist.

2. Verfahren zum partiellen Aufreinigen eines hydrophilen rekombinanten Proteins nach Anspruch 1, wobei es sich bei dem lösenden Lösemittel um eine oder mehrere Arten eines aprotischen polaren Lösemittels handelt, das aus der Gruppe bestehend aus Dimethylsulfoxid, N,N-Dimethylformamid, N,N-Dimethylacetamid, 1,3-Dimethyl-2-Imidazolidon und N-Methyl-2-pyrrolidon ausgewählt ist.

3. Verfahren zum partiellen Aufreinigen eines hydrophilen rekombinanten Proteins nach Anspruch 1 oder 2, wobei es sich bei dem lösenden Lösemittel um ein Lösemittel handelt, das durch Zusatz eines anorganischen Salzes zu dem aprotischen polaren Lösemittel erhalten wird.

4. Verfahren zum partiellen Aufreinigen eines hydrophilen rekombinanten Proteins nach einem der Ansprüche 1 bis 3, wobei die Abtrennung der unlöslichen Substanz durch Filtration und/oder Zentrifugation durchgeführt wird.

5. Verfahren zum partiellen Aufreinigen eines hydrophilen rekombinanten Proteins nach einem der Ansprüche 1 bis 4, wobei die Menge des lösenden Lösemittels, die zugesetzt wird, das 0,5-Fache oder mehr der Masse des Wirts, ausgedrückt als Verhältnis Volumen (mL)/Masse (g), beträgt.

6. Verfahren zum partiellen Aufreinigen eines hydrophilen rekombinanten Proteins nach einem der Ansprüche 1 bis 5, wobei die Lösung einer Lösemittelsubstitution unterzogen wird, wobei ein wässriges Lösemittel verwendet wird.

7. Verfahren zum partiellen Aufreinigen eines hydrophilen rekombinanten Proteins nach Anspruch 6, wobei die Lösung nach der Lösemittelsubstitution durch wenigstens eines, ausgewählt aus der Gruppe bestehend aus einer Säule, Filtration und Zentrifugation, weiterverarbeitet wird.

8. Verfahren zum partiellen Aufreinigen eines hydrophilen rekombinanten Proteins nach einem der Ansprüche 1 bis 7, wobei es sich bei dem hydrophilen rekombinanten Protein um ein Protein handelt, das aus der Gruppe bestehend aus einem rekombinanten Spinnenseidenprotein, einem rekombinanten Resilin und einem rekombinanten Collagen ausgewählt ist.

## Revendications

1. Procédé pour purifier partiellement une protéine recombinante hydrophile provenant d'un hôte exprimant une protéine recombinante hydrophile,
le procédé comprenant une étape d'acquisition de solution dans laquelle un solvant dissolvant est ajouté à l'hôte exprimant une protéine recombinante hydrophile afin de dissoudre la cellule hôte et une substance insoluble en est séparée afin d'obtenir une solution,
dans lequel la protéine recombinante hydrophile a un indice d'hydropathie de 0 ou moins, et
le solvant dissolvant est un solvant polaire aprotique ayant un moment dipolaire de 3,0 D ou plus.

2. Procédé pour purifier partiellement une protéine recombinante hydrophile selon la revendication 1, dans lequel le solvant dissolvant est une ou plusieurs sortes de solvant polaire aprotique sélectionné à partir du groupe constitué par du sulfoxyde diméthylique, du N,N-éthylformamide, du N,N-diméthylacétamide, de la 1,3-diméthyl-2-imidazolidone et de la N-méthyl-2-pyrrolidone.

3. Procédé pour purifier partiellement une protéine recombinante hydrophile selon la revendication 1 ou 2, dans lequel le solvant dissolvant est un solvant obtenu en ajoutant un sel inorganique au solvant polaire aprotique.

4. Procédé pour purifier partiellement une protéine recombinante hydrophile selon l'une quelconque des revendications 1 à 3, dans lequel la séparation de la substance insoluble est effectuée par filtration et/ou centrifugation.

5. Procédé pour purifier partiellement une protéine recombinante hydrophile selon l'une quelconque des revendications 1 à 4, dans lequel la quantité du solvant dissolvant qui est ajoutée est 0,5 fois ou plus la masse de l'hôte, par le rapport volume (mL)/masse (g).

6. Procédé pour purifier partiellement une protéine recombinante hydrophile selon l'une quelconque des revendications 1 à 5, dans lequel la solution est soumise à une substitution de solvant en utilisant un solvant aqueux.

7. Procédé pour purifier partiellement une protéine recombinante hydrophile selon la revendication 6, dans lequel la solution après substitution de solvant est traitée en outre par au moins un processus sélectionné dans le groupe constitué par une colonne, une filtration et une centrifugation.

8. Procédé pour purifier partiellement une protéine recombinante hydrophile selon l'une quelconque des revendications 1 à 7, dans lequel la protéine recombinante hydrophile est une protéine sélectionnée dans le groupe constitué par une protéine de soie d'araignée recombinante, une résiline recombinante et un collagène recombinant.


### SEQUENCE LISTING

<110> Spiber Inc.
<120> THE PARTIAL PURIFICATION METHOD OF HYDROPHILIC PROTEINS
<130> 42.34.124573
<140> EP13868047.4
   <141> 2013-12-18
<150> JP 2012-285392
   <151> 2012-12-27
<150> JP2013-93926
   <151> 2013-04-26
<160> 15
<170> PatentIn version 3.3
<210> 1
   <211> 136
   <212> PRT
   <213> Artificial
<220>
   <223> ADF3Kai-small-M
<400> 1
   Met His His His His His His His His His His Ser Ser Gly Ser Ser
   1               5                   10                  15
   Leu Glu Val Leu Phe Gln Gly Pro Ala Arg Ala Gly Ser Gly Gln Gln
               20                  25                  30
   Gly Pro Gly Gln Gln Gly Pro Gly Gln Gln Gly Pro Gly Gln Gln Gly
           35                  40                  45
   Pro Tyr Gly Pro Gly Ala Ser Ala Ala Ala Ala Ala Ala Gly Gly Tyr
       50                  55                  60
   Gly Pro Gly Ser Gly Gln Gln Gly Pro Ser Gln Gln Gly Pro Gly Gln
   65                  70                  75                  80
   Gln Gly Pro Gly Gly Gln Gly Pro Tyr Gly Pro Gly Ala Ser Ala Ala
                   85                  90                  95
   Ala Ala Ala Ala Gly Gly Tyr Gly Pro Gly Ser Gly Gln Gln Gly Pro
               100                 105                 110
   Gly Gln Gln Gly Pro Gly Gln Gln Gly Pro Gly Gly Gln Gly Pro Tyr
           115                 120                 125
   Gly Pro Gly Ala Ala Ser Ala Ala
       130                 135
<210> 2
   <211> 542
   <212> PRT
   <213> Artificial
<220>
   <223> ADF3Kai-NN
<400> 2
   Met His His His His His His His His His His Ser Ser Gly Ser Ser
   1               5                   10                  15
   Leu Glu Val Leu Phe Gln Gly Pro Ala Arg Ala Gly Ser Gly Gln Gln
               20                  25                  30
   Gly Pro Gly Gln Gln Gly Pro Gly Gln Gln Gly Pro Gly Gln Gln Gly
           35                  40                  45
   Pro Tyr Gly Pro Gly Ala Ser Ala Ala Ala Ala Ala Ala Gly Gly Tyr
       50                  55                  60
   Gly Pro Gly Ser Gly Gln Gln Gly Pro Ser Gln Gln Gly Pro Gly Gln
   65                  70                  75                  80
   Gln Gly Pro Gly Gly Gln Gly Pro Tyr Gly Pro Gly Ala Ser Ala Ala
                   85                  90                  95
   Ala Ala Ala Ala Gly Gly Tyr Gly Pro Gly Ser Gly Gln Gln Gly Pro
               100                 105                 110
   Gly Gly Gln Gly Pro Tyr Gly Pro Gly Ser Ser Ala Ala Ala Ala Ala
           115                 120                 125
   Ala Gly Gly Asn Gly Pro Gly Ser Gly Gln Gln Gly Ala Gly Gln Gln
       130                 135                 140
   Gly Pro Gly Gln Gln Gly Pro Gly Ala Ser Ala Ala Ala Ala Ala Ala
   145                 150                 155                 160
   Gly Gly Tyr Gly Pro Gly Ser Gly Gln Gln Gly Pro Gly Gln Gln Gly
                   165                 170                 175
   Pro Gly Gly Gln Gly Pro Tyr Gly Pro Gly Ala Ser Ala Ala Ala Ala
               180                 185                 190
   Ala Ala Gly Gly Tyr Gly Pro Gly Ser Gly Gln Gly Pro Gly Gln Gln
               195                 200                 205
   Gly Pro Gly Gly Gln Gly Pro Tyr Gly Pro Gly Ala Ser Ala Ala Ala
       210                 215                 220
   Ala Ala Ala Gly Gly Tyr Gly Pro Gly Ser Gly Gln Gln Gly Pro Gly
   225                 230                 235                 240
   Gln Gln Gly Pro Gly Gln Gln Gly Pro Gly Gly Gln Gly Pro Tyr Gly
                   245                 250                 255
   Pro Gly Ala Ser Ala Ala Ala Ala Ala Ala Gly Gly Tyr Gly Pro Gly
               260                 265                 270
   Tyr Gly Gln Gln Gly Pro Gly Gln Gln Gly Pro Gly Gly Gln Gly Pro
           275                 280                 285
   Tyr Gly Pro Gly Ala Ser Ala Ala Ser Ala Ala Ser Gly Gly Tyr Gly
       290                 295                 300
   Pro Gly Ser Gly Gln Gln Gly Pro Gly Gln Gln Gly Pro Gly Gly Gln
   305                 310                 315                 320
   Gly Pro Tyr Gly Pro Gly Ala Ser Ala Ala Ala Ala Ala Ala Gly Gly
                   325                 330                 335
   Tyr Gly Pro Gly Ser Gly Gln Gln Gly Pro Gly Gln Gln Gly Pro Gly
               340                 345                 350
   Gln Gln Gly Pro Gly Gln Gln Gly Pro Gly Gly Gln Gly Pro Tyr Gly
           355                 360                 365
   Pro Gly Ala Ser Ala Ala Ala Ala Ala Ala Gly Gly Tyr Gly Pro Gly
       370                 375                 380
   Ser Gly Gln Gln Gly Pro Gly Gln Gln Gly Pro Gly Gln Gln Gly Pro
   385                 390                 395                 400
   Gly Gln Gln Gly Pro Gly Gln Gln Gly Pro Gly Gln Gln Gly Pro Gly
                   405                 410                 415
   Gln Gln Gly Pro Gly Gln Gln Gly Pro Gly Gln Gln Gly Pro Gly Gly
               420                 425                 430
   Gln Gly Ala Tyr Gly Pro Gly Ala Ser Ala Ala Ala Gly Ala Ala Gly
           435                 440                 445
   Gly Tyr Gly Pro Gly Ser Gly Gln Gln Gly Pro Gly Gln Gln Gly Pro
   450 455 460
   Gly Gln Gln Gly Pro Gly Gln Gln Gly Pro Gly Gln Gln Gly Pro Gly
   465 470 475 480
   Gln Gln Gly Pro Gly Gln Gln Gly Pro Gly Gln Gln Gly Pro Tyr Gly
   485 490 495
   Pro Gly Ala Ser Ala Ala Ala Ala Ala Ala Gly Gly Tyr Gly Pro Gly
   500 505 510
   Ser Gly Gln Gln Gly Pro Gly Gln Gln Gly Pro Gly Gln Gln Gly Pro
   515 520 525
   Gly Gly Gln Gly Pro Tyr Gly Pro Gly Ala Ala Ser Ala Ala
   530 535 540
<210> 3
   <211> 24
   <212> PRT
   <213> Artificial
<220>
   <223> His tag
<400> 3
   Met His His His His His His His His His His Ser Ser Gly Ser Ser
   1 5 10 15
   Leu Glu Val Leu Phe Gln Gly Pro
   20
<210> 4
   <211> 408
   <212> DNA
   <213> Artificial
<220>
   <223> ADF3Kai-small-M
<400> 4
   atgcaccacc accatcacca tcatcatcac cactcatcag gttcaagcct tgaagtatta 60
   tttcagggac ccgcacgcgc aggctcaggg cagcagggtc cgggtcagca ggggcctgga 120
   caacaaggac cagggcaaca ggggccctac gggccaggag cttcagcagc agctgcggct 180
   gccggtggtt atggacccgg cagtggccag caaggcccca gccaacaagg tccaggacag 240
   cagggcccag gtggtcaagg accttacgga cctggtgcga gcgcggcagc ggccgccgcg 300
   ggaggttacg gccccggtag cggtcaacag ggtcctggtc agcaaggtcc cggccaacag 360
   ggaccgggcg gccaagggcc ttatgggccg ggcgcagctt ccgcagcg 408
<210> 5
   <211> 1626
   <212> DNA
   <213> Artificial
<220>
   <223> ADF3Kai-NN
<400> 5
   atgcatcacc atcatcatca tcaccaccac cattcctcgg gctcatcctt ggaagtgtta      60
   tttcaaggac cagcacgagc cggttcggga caacaagggc ctggccagca gggcccaggt     120
   caacaagggc caggacagca gggtccttat gggcccggcg caagcgcagc agctgcggcc     180
   gctggtggct atggtcctgg ctccggtcaa cagggccctt cgcaacaagg tcccgggcag     240
   caaggtcctg gtggccaggg tccctacggg ccgggggcga gtgcggcagc agccgctgca     300
   ggcggttatg gtccaggaag cggacagcaa ggtccgggag gtcaaggtcc gtatggccca     360
   ggctctagcg cggctgccgc tgccgcgggt ggcaacggac cagggagcgg acaacagggc     420
   gcgggacaac agggtccagg acagcaaggc ccaggggcgt cggcggctgc agcggcggcc     480
   ggaggctatg gacccggctc aggacaacag ggaccgggtc aacaaggacc cggtggccaa     540
   ggcccctatg gcccgggcgc cagcgcggcc gcagccgccg cgggcgggta cggccccggt     600
   agcggccagg gaccaggtca gcaggggcca ggaggtcagg gcccatacgg tccgggcgca     660
   tccgcggcgg cggcagcggc aggtggctac ggtcccggaa gcggccaaca ggggccaggg     720
   caacaaggac caggacaaca aggtcctggg ggccaaggac cgtatggacc aggagcatca     780
   gctgcagccg cggcagctgg cggttacggt ccaggctacg gccagcaggg tccgggtcag     840
   cagggaccgg gaggccaggg gccttatggc cctggcgctt ccgcagccag tgccgcttct     900
   ggaggatacg ggccgggaag cggtcagcaa ggccctggcc aacaaggacc tggaggccaa     960
   gggccctacg gcccaggagc ctcggcagcc gcagctgccg caggtgggta tgggccaggt    1020
   agcgggcaac aagggccggg tcagcaagga ccggggcaac agggacctgg gcagcaagga    1080
   cccgggggtc aaggcccgta cggacctggt gcgtctgcag ctgctgctgc ggctggtgga    1140
   tatggtccgg gatcggggca gcagggtccc ggtcagcagg gccctggtca gcaagggcca    1200
   ggccaacagg gacccggaca acaaggcccg ggtcaacagg gtcctggaca gcaggggccg    1260
   ggccaacaag gccctgggca acagggtccg gggggacagg gggcctatgg gcctggcgca    1320
   tctgccgccg ctggcgcagc cggtgggtac gggcctgggt caggtcaaca ggggcctggt    1380
   caacaaggcc ccgggcaaca gggccccggc cagcaaggtc cagggcagca gggcccggga    1440
   cagcaagggc ctggacaaca ggggcccgga cagcagggac cttacgggcc cggtgcgagc    1500
   gcagcggccg ccgccgcagg gggatatggc cccggatcgg gccagcaggg accaggccag    1560
   caaggacctg gccaacaggg cccggggggt caggggccgt atggtcccgg cgctgcaagt     1620
   gctgca                                                                1626
<210> 6
   <211> 318
   <212> PRT
   <213> Artificial
<220>
   <223> ADF4Kai-W
<400> 6
   Met His His His His His His His His His His Ser Ser Gly Ser Ser
   1               5                   10                  15
   Leu Glu Val Leu Phe Gln Gly Pro Ala Gly Ser Ser Ala Ala Ala Ala
               20                  25                  30
   Ala Ala Ala Ser Gly Ser Gly Gly Tyr Gly Pro Glu Asn Gln Gly Pro
           35                  40                  45
   Ser Gly Pro Val Ala Tyr Gly Pro Gly Gly Pro Val Ser Ser Ala Ala
       50                  55                  60
   Ala Ala Ala Ala Ala Gly Ser Gly Pro Gly Gly Tyr Gly Pro Glu Asn
   65                  70                  75                  80
   Gln Gly Pro Ser Gly Pro Gly Gly Tyr Gly Pro Gly Gly Ser Gly Ser
                   85                  90                  95
   Ser Ala Ala Ala Ala Ala Ala Ala Ala Ser Gly Pro Gly Gly Tyr Gly
               100                 105                 110
   Pro Gly Ser Gln Gly Pro Ser Gly Pro Gly Gly Ser Gly Gly Tyr Gly
           115                 120                 125
   Pro Gly Ser Gln Gly Ala Ser Gly Pro Gly Gly Pro Gly Ala Ser Ala
       130                 135                 140
   Ala Ala Ala Ala Ala Ala Ala Ala Ala Ser Gly Pro Gly Gly Tyr Gly
   145                 150                 155                 160
   Pro Gly Ser Gln Gly Pro Ser Gly Pro Gly Ala Tyr Gly Pro Gly Gly
                   165                 170                 175
   Pro Gly Ser Ser Ala Ala Ala Ala Ala Ala Ala Ala Ser Gly Pro Gly
               180                 185                 190
   Gly Tyr Gly Pro Gly Ser Gln Gly Pro Ser Gly Pro Gly Val Tyr Gly
           195                 200                 205
   Pro Gly Gly Pro Gly Ser Ser Ala Ala Ala Ala Ala Ala Ala Gly Ser
       210                 215                 220
   Gly Pro Gly Gly Tyr Gly Pro Glu Asn Gln Gly Pro Ser Gly Pro Gly
   225                 230                 235                 240
   Gly Tyr Gly Pro Gly Gly Ser Gly Ser Ser Ala Ala Ala Ala Ala Ala
                   245                 250                 255
   Ala Ala Ser Gly Pro Gly Gly Tyr Gly Pro Gly Ser Gln Gly Pro Ser
               260                 265                 270
   Gly Pro Gly Gly Ser Gly Gly Tyr Gly Pro Gly Ser Gln Gly Gly Ser
           275                 280                 285
   Gly Pro Gly Ala Ser Ala Ala Ala Ala Ala Ala Ala Ala Ser Gly Pro
       290                 295                 300
   Gly Gly Tyr Gly Pro Gly Ser Gln Gly Pro Ser Gly Pro Gly
   305                 310                 315
<210> 7
   <211> 954
   <212> DNA
   <213> Artificial
<220>
   <223> ADF4Kai-W
<400> 7
   atgcaccatc atcatcacca tcaccaccat catagcagtg gatcttccct cgaagtactc      60
   tttcaaggtc cagcaggtag tagcgctgcc gcagctgcag cagcttcagg ttccggtggc     120
   tacgggccag aaaatcaggg tccgagcggt ccggttgcgt atggcccagg tggccctgtg     180
   agcagcgcag cagcagccgc agcagctggt agcggacccg ggggctacgg ccctgagaat      240
   caaggaccga gtggcccggg gggttatggt cctggaggta gtgggagcag cgcggcggct     300
   gcagcagcgg cggcgagtgg gccaggtgga tatgggccag gctcccaggg gcctagtggc     360
   cctggtggtt caggagggta tggaccagga tcacagggcg catccggacc tggcggcccg     420
   ggtgcaagtg ctgcggcggc cgcagctgct gctgccgcca gtggaccagg cgggtatggc     480
   cctggatccc aaggtccatc gggaccagga gcatacgggc ctgggggacc aggctccagt     540
   gcggccgcgg cggcggcggc agcctccggt ccaggaggtt atggaccggg aagtcaaggc     600
   ccttctggac ctggagtgta cggtccaggc ggaccgggtt cgagtgctgc agcggcagcg     660
   gccgcaggtt cgggccccgg aggctacgga ccagagaatc aggggccgtc tggcccggga     720
   ggctatggtc cgggcggctc cggttccagt gcagcggctg ccgcggctgc ggcgtcaggc     780
   cctggcggtt atggccccgg tagccaaggt cccagtggtc cgggaggaag cggcggttac     840
   ggtccgggca gccaaggtgg ctcaggtcct ggtgccagcg cggctgccgc agcggcagct     900
   gcgtcaggtc caggtggtta tgggccgggt agccaggggc catctggtcc tggg           954
<210> 8
   <211> 467
   <212> PRT
   <213> Artificial
<220>
   <223> Flag92-short2-UU
<400> 8
   Met His His His His His His His His His His Ser Ser Gly Ser Ser
   1               5                   10                  15
   Leu Glu Val Leu Phe Gln Gly Pro Gly Ala Gly Gly Ser Gly Pro Gly
               20                  25                  30
   Gly Ala Gly Pro Gly Gly Val Gly Pro Gly Gly Ser Gly Pro Gly Gly
           35                  40                  45
   Val Gly Pro Gly Gly Ser Gly Pro Gly Gly Val Gly Pro Gly Gly Ser
       50                  55                  60
   Gly Pro Gly Gly Val Gly Pro Gly Gly Ala Gly Gly Pro Tyr Gly Pro
   65                  70                  75                  80
   Gly Gly Ser Gly Pro Gly Gly Ala Gly Gly Ala Gly Gly Pro Gly Gly
                   85                  90                  95
   Ala Tyr Gly Pro Gly Gly Ser Tyr Gly Pro Gly Gly Ser Gly Gly Pro
               100                 105                 110
   Gly Gly Ala Gly Gly Pro Tyr Gly Pro Gly Gly Glu Gly Pro Gly Gly
           115                 120                 125
   Ala Gly Gly Pro Tyr Gly Pro Gly Gly Ala Gly Gly Pro Tyr Gly Pro
       130                 135                 140
   Gly Gly Ala Gly Gly Pro Tyr Gly Pro Gly Gly Glu Gly Gly Pro Tyr
   145                 150                 155                 160
   Gly Pro Gly Gly Ser Tyr Gly Pro Gly Gly Ala Gly Gly Pro Tyr Gly
                   165                 170                 175
   Pro Gly Gly Pro Tyr Gly Pro Gly Gly Glu Gly Pro Gly Gly Ala Gly
               180                 185                 190
   Gly Pro Tyr Gly Pro Gly Gly Val Gly Pro Gly Gly Gly Gly Pro Gly
           195                 200                 205
   Gly Tyr Gly Pro Gly Gly Ala Gly Pro Gly Gly Tyr Gly Pro Gly Gly
       210                 215                 220
   Ser Gly Pro Gly Gly Tyr Gly Pro Gly Gly Ser Gly Pro Gly Gly Tyr
   225                 230                 235                 240
   Gly Pro Gly Gly Ser Gly Pro Gly Gly Tyr Gly Pro Gly Gly Ser Gly
                   245                 250                 255
   Pro Gly Gly Tyr Gly Pro Gly Gly Ser Gly Pro Gly Gly Ser Gly Pro
               260                 265                 270
   Gly Gly Tyr Gly Pro Gly Gly Ser Gly Pro Gly Gly Ser Gly Pro Gly
           275                 280                 285
   Gly Tyr Gly Pro Gly Gly Ser Gly Pro Gly Gly Tyr Gly Pro Gly Gly
       290                 295                 300
   Ser Gly Pro Gly Gly Ser Gly Pro Gly Gly Tyr Gly Pro Gly Gly Ser
   305                 310                 315                 320
   Gly Pro Gly Gly Ser Gly Pro Gly Gly Tyr Gly Pro Gly Gly Ser Gly
                   325                 330                 335
   Pro Gly Gly Phe Gly Pro Gly Gly Phe Gly Pro Gly Gly Ser Gly Pro
               340                 345                 350
   Gly Gly Tyr Gly Pro Gly Gly Ser Gly Pro Gly Gly Ala Gly Pro Gly
           355                 360                 365
   Gly Val Gly Pro Gly Gly Phe Gly Pro Gly Gly Ala Gly Pro Gly Gly
       370                 375                 380
   Ala Gly Pro Gly Gly Ala Gly Pro Gly Gly Ala Gly Pro Gly Gly Ala
   385                 390                 395                 400
   Gly Pro Gly Gly Ala Gly Pro Gly Gly Ala Gly Pro Gly Gly Ala Gly
                   405                 410                 415
   Pro Gly Gly Ala Gly Gly Ala Gly Gly Ala Gly Gly Ala Gly Gly Ser
               420                     425                     430
   Gly Gly Ala Gly Gly Ser Gly Gly Thr Thr Ile Ile Glu Asp Leu Asp
           435                     440                     445
   Ile Thr Ile Asp Gly Ala Asp Gly Pro Ile Thr Ile Ser Glu Glu Leu
       450                     455                     460
   Thr Ile Ser
   465
<210> 9
   <211> 1401
   <212> DNA
   <213> Artificial
<220>
   <223> Flag92-short2-UU
<400> 9
   atgcatcacc atcatcatca tcaccaccac cattcctcgg gctcatcctt ggaagtgtta      60
   tttcaaggac caggagctgg agggtccggt ccgggcggcg ctggtccggg aggagtgggt     120
   ccgggtggtt ctggacccgg tggagtaggt ccgggcggtt ccggtccggg aggcgtcgga     180
   cctggtggat caggacctgg aggtgtaggt cctggtggcg cgggtggccc ttacggccct     240
   gggggctctg gcccaggagg cgctggtggg gcaggagggc ctggtggggc gtatggtccc     300
   gggggttcat acggcccagg gggttcagga ggtccaggag gtgcaggggg accatacgga     360
   ccgggcggtg agggaccagg gggcgctggc ggaccctatg gacctggcgg agctggaggt     420
   ccctacgggc caggcggggc tggaggaccg tatggaccgg gtggagaagg tggtccgtac     480
   ggcccgggcg ggtcttacgg accaggtggc gcaggaggtc cttatgggcc gggtggacca     540
   tacggtccag gcggagaggg tccaggtggg gcaggtgggc cttatggccc gggaggagtt     600
   ggtccaggcg gcggtggtcc gggcgggtat ggcccgggtg gcgcaggtcc aggcggatat     660
   ggaccaggtg gctctggacc cggagggtac gggccgggtg gttcagggcc tggaggttat     720
   ggtccgggag gtagtggacc aggaggttat gggcctgggg gttcagggcc aggtggttat     780
   ggtccaggag gatcaggtcc aggtggaagt ggtccagggg gttatggccc tggtggtagc     840
   ggcccaggtg gcagtggacc gggaggatat ggcccaggag gttcaggtcc cggtggatac     900
   ggaccgggtg gatctggacc aggcggttct ggtccgggag gttacggtcc tgggggtagt     960
   ggtcccggag gtagtggacc aggtggatat ggaccaggag ggtctggacc cggaggtttt    1020
   ggtccaggcg gatttggtcc tggtggttct gggcctggtg ggtatggtcc tggcggatct    1080
   ggtccaggtg gggcaggacc tggtggagtt ggacctgggg gttttggacc cggtggtgcg    1140
   ggaccagggg gtgcaggtcc tggtggcgct ggtcctggag gcgctgggcc aggcggtgct    1200
   ggacctggag gagcaggacc aggtggtgct ggccccggtg gcgcaggccc tggaggtgct    1260
   ggtggagctg gtggtgcagg tggagcaggt ggttcaggag gagctggtgg ttcaggtgga    1320
   actactataa tcgaagacct tgatattacg attgatggag cagatggtcc catcactatt    1380
   tcggaagaat tgactatttc a                                              1401
<210> 10
   <211> 310
   <212> PRT
   <213> Artificial Sequence
<220>
   <223> Drosophila-Resilin-Kai
<400> 10
   Met His His His His His His Pro Glu Pro Pro Val Asn Ser Tyr Leu
   1               5                   10                  15
   Pro Pro Ser Asp Ser Tyr Gly Ala Pro Gly Gln Ser Gly Pro Gly Gly
               20                  25                  30
   Arg Pro Ser Asp Ser Tyr Gly Ala Pro Gly Gly Gly Asn Gly Gly Arg
           35                  40                  45
   Pro Ser Asp Ser Tyr Gly Ala Pro Gly Gln Gly Gln Gly Gln Gly Gln
       50                  55                  60
   Gly Gln Gly Gly Tyr Ala Gly Lys Pro Ser Asp Ser Tyr Gly Ala Pro
   65                  70                  75                  80
   Gly Gly Gly Asp Gly Asn Gly Gly Arg Pro Ser Ser Ser Tyr Gly Ala
                   85                  90                  95
   Pro Gly Gly Gly Asn Gly Gly Arg Pro Ser Asp Thr Tyr Gly Ala Pro
               100                 105                 110
   Gly Gly Gly Asn Gly Gly Arg Pro Ser Asp Thr Tyr Gly Ala Pro Gly
           115                 120                 125
   Gly Gly Gly Asn Gly Asn Gly Gly Arg Pro Ser Ser Ser Tyr Gly Ala
       130                 135                 140
   Pro Gly Gln Gly Gln Gly Asn Gly Asn Gly Gly Arg Pro Ser Ser Ser
   145                 150                 155                 160
   Tyr Gly Ala Pro Gly Gly Gly Asn Gly Gly Arg Pro Ser Asp Thr Tyr
                   165                 170                 175
   Gly Ala Pro Gly Gly Gly Asn Gly Gly Arg Pro Ser Asp Thr Tyr Gly
               180                 185                 190
   Ala Pro Gly Gly Gly Asn Asn Gly Gly Arg Pro Ser Ser Ser Tyr Gly
           195                 200                 205
   Ala Pro Gly Gly Gly Asn Gly Gly Arg Pro Ser Asp Thr Tyr Gly Ala
       210                 215                 220
   Pro Gly Gly Gly Asn Gly Asn Gly Ser Gly Gly Arg Pro Ser Ser Ser
   225                 230                 235                 240
   Tyr Gly Ala Pro Gly Gln Gly Gln Gly Gly Phe Gly Gly Arg Pro Ser
                   245                 250                 255
   Asp Ser Tyr Gly Ala Pro Gly Gln Asn Gln Lys Pro Ser Asp Ser Tyr
               260                 265                 270
   Gly Ala Pro Gly Ser Gly Asn Gly Asn Gly Gly Arg Pro Ser Ser Ser
           275                 280                 285
   Tyr Gly Ala Pro Gly Ser Gly Pro Gly Gly Arg Pro Ser Asp Ser Tyr
       290                 295                 300
   Gly Pro Pro Ala Ser Gly
   305                 310
<210> 11
   <211> 930
   <212> DNA
   <213> Artificial Sequence
<220>
   <223> Drosophila-Resilin-Kai
<400> 11
   atgcatcatc accaccatca tccggaacct cctgtcaaca gctatttgcc ccctagtgat     60
   tcttacggag caccaggtca gtctggacca ggcgggagac cttctgacag ttacggcgct    120
   cctggtggcg gcaatggtgg gaggccatct gatagctacg gagccccagg ccaagggcag    180
   ggtcagggcc aaggtcaagg tggctacgct ggtaaaccgt cggactctta tggtgcacct    240
   ggcggggggg acggaaacgg tggcagaccc agcagttcct atggggcacc cggtggaggc    300
   aatgggggcc gacctagcga cacttacggg gcccccggcg gcgggaacgg tgggcgtccc    360
   agcgatacct acggtgcccc gggtggggga gggaacggca acgggggaag gcctagttct    420
   agttatggcg cgccgggcca gggacaaggg aatggtaacg gagggcgccc atccagctcg    480
   tatggcgctc ccgggggtgg taatgggggt cgcccttcag atacatacgg ggctccaggg    540
   gggggtaacg gcggacggcc ctcggacacc tatggagctc cgggcggagg aaataatgga    600
   ggccgcccaa gttcatctta tggagcgccc ggtggtggaa acggcggtag accgtcagac    660
   acgtatgggg cgcctggagg cggtaatggc aacggttcgg gaggtaggcc gagctcaagc    720
   tatggtgcgc cagggcaagg acagggcggt tttggcggaa gaccatccga tagttacggt    780
   gctcctgggc agaaccaaaa accttcggat tcatacggcg cccctggtag tgggaatgga    840
   aatggcggtc gtccatcgtc ctcctacggc gcaccgggaa gcggtcccgg aggtcgaccg    900
   agtgactcgt acggtccacc tgcaagtggt                                     930
<210> 12
   <211> 7
   <212> PRT
   <213> Artificial Sequence
<220>
   <223> HisTag6
<400> 12
   Met His His His His His His
   1               5
<210> 13
   <211> 252
   <212> PRT
   <213> Artificial Sequence
<220>
   <223> Collagen-type4-Kai
<400> 13
   Met His His His His His His Ser Ser Gly Ser Ser Lys Asp Gly Val
   1               5                   10                  15
   Pro Gly Phe Pro Gly Ser Glu Gly Val Lys Gly Asn Arg Gly Phe Pro
               20                  25                  30
   Gly Leu Met Gly Glu Asp Gly Ile Lys Gly Gln Lys Gly Asp Ile Gly
           35                  40                  45
   Pro Pro Gly Phe Arg Gly Pro Thr Glu Tyr Tyr Asp Thr Tyr Gln Glu
       50                  55                  60
   Lys Gly Asp Glu Gly Thr Pro Gly Pro Pro Gly Pro Arg Gly Ala Arg
   65                  70                  75                  80
   Gly Pro Gln Gly Pro Ser Gly Pro Pro Gly Val Pro Gly Ser Pro Gly
                   85                  90                  95
   Ser Ser Arg Pro Gly Leu Arg Gly Ala Pro Gly Trp Pro Gly Leu Lys
               100                 105                 110
   Gly Ser Lys Gly Glu Arg Gly Arg Pro Gly Lys Asp Ala Met Gly Thr
           115                 120                 125
   Pro Gly Ser Pro Gly Cys Ala Gly Ser Pro Gly Leu Pro Gly Ser Pro
       130                 135                 140
   Gly Pro Pro Gly Pro Pro Gly Asp Ile Val Phe Arg Lys Gly Pro Pro
   145                 150                 155                 160
   Gly Asp His Gly Leu Pro Gly Tyr Leu Gly Ser Pro Gly Ile Pro Gly
                   165                 170                 175
   Val Asp Gly Pro Lys Gly Glu Pro Gly Leu Leu Cys Thr Gln Cys Pro
               180                 185                 190
   Tyr Ile Pro Gly Pro Pro Gly Leu Pro Gly Leu Pro Gly Leu His Gly
           195                 200                 205
   Val Lys Gly Ile Pro Gly Arg Gln Gly Ala Ala Gly Leu Lys Gly Ser
       210                 215                 220
   Pro Gly Ser Pro Gly Asn Thr Gly Leu Pro Gly Phe Pro Gly Phe Pro
   225                 230                 235                 240
   Gly Ala Gln Gly Asp Pro Gly Leu Lys Gly Glu Lys
                   245                 250
<210> 14
   <211> 756
   <212> DNA
   <213> Artificial Sequence
<220>
   <223> Collagen-type4-Kai
<400> 14
   atgcaccatc atcaccacca tagctctgga agttcaaaag acggagtacc tgggtttcca     60
   ggttcggagg gggttaaagg aaatcgagga ttccctgggc tactgggaga agacggcata    120
   aaaggtcaga aaggcgacat aggtcctccc ggatttagag gccccacaga atactacgac    180
   acgtatcaag aaaagggaga tgagggcact ccggggccgc ccggcccacg aggggctcga    240
   ggtccccaag gaccgtctgg accaccaggc gtgcctggct cccccggcag ttcccgccca    300
   ggattacgcg gagctccagg atggccaggg ttgaaaggta gtaaaggtga gcgtggccgt    360
   cctgggaaag atgcggttgg cacaccgggg tctcctgggt gcgcgggtag tcccggtctt    420
   ccgggttctc cgggccctcc tggacctcca ggggacattg tgttcaggaa gggtccacct    480
   ggcgatcatg gacttcccgg ctatctcgga agcccgggga tacctggggt agatgggcct    540
   aaaggggaac caggccttct ttgtacccag tgtccgtata taccgggtcc gcccggttta    600
   ccaggtttgc cagggctgca tggtgtcaag ggcattcctg gtcgtcaggg cgcagcgggt    660
   ctcaaaggat cacccgggag tccgggcaac accggcttac ccggctttcc gggttttcct    720
   ggcgcgcaag gtgaccctgg attaaaagga gagaag                              756
<210> 15
   <211> 12
   <212> PRT
   <213> Artificial Sequence
<220>
   <223> HisTag6-II
<400> 15
   Met His His His His His His Ser Ser Gly Ser Ser
   1               5                   10